(19) Europäisches Patentamt
European Patent Office
Office européen des brevets

(11) Publication number: **0 013 153**
**B1**

(12) # EUROPEAN PATENT SPECIFICATION

(45) Date of publication of patent specification: **22.12.82**

(21) Application number: **79302998.4**

(22) Date of filing: **20.12.79**

(51) Int. Cl.³: **C 07 D 213/80,**
C 07 D 213/74,
A 61 K 31/44,
A 61 K 31/455

(54) **6-Amino pyridine carboxylic acid derivatives, a process for their preparation and pharmaceutical compositions containing them.**

(30) Priority: **27.12.78 GB 5006078**

(43) Date of publication of application:
**09.07.80 Bulletin 80/14**

(45) Publication of the grant of the patent:
**22.12.82 Bulletin 82/51**

(84) Designated Contracting States:
**CH DE FR GB IT NL**

(56) References cited:
**DE - A - 2 803 592**
**US - A - 2 194 567**

(73) Proprietor: **BEECHAM GROUP PLC**
**Beecham House Great West Road**
**Brentford Middlesex (GB)**

(72) Inventor: **Hindley, Richard Mark**
**19 Cockshot Road**
**Reigate Surrey (GB)**

(74) Representative: **Cresswell, Thomas Anthony et al,**
**European Patent Attorney Beecham**
**Pharmaceuticals Great Burgh Yew Tree Bottom**
**Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 013 153 B1

6-Amino pyridine carboxylic acid derivatives, a process for their preparation and pharmaceutical compositions containing them

This invention relates to a series of novel nicotinic acid derivatives, to a method for their preparation and to their use as hypolipidaemic agents.

USP 2 194 567 discloses 2-amino-5-aminoalkoxycarbonyl piperidine derivatives which are stated to have anaesthetic activity.

Accordingly the present invention provides a compound of formula (I):—

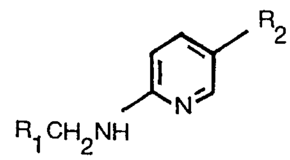

(I)

wherein $R_1CH_2$— is a $C_9$ to $C_{25}$ alkyl or alkenyl group which is optionally branched or unbranched or includes a mono-, bi- or polycycloalkyl or — cycloalkenyl moiety and $R_2$ is —$CO_2H$ or —$CH_2CO_2H$ or a pharmaceutically acceptable salt thereof or a pharmaceutically acceptable group, which is convertible in the human body into a —$CO_2H$ or —$CH_2CO_2H$ group, selected from a pharmaceutically acceptable ester

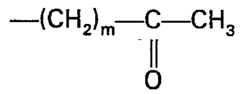　　　$-(CH_2)_n-CH_2OH$　　　$-(CH_2)_p-CO_2H$

wherein m is an integer up to 5, n is zero or an integer up to 5 and p is an integer from 2 to 6.

Most suitably $R_1CH_2$— does not contain more than 20 carbon atoms. Examples of suitable unbranched alkyl groups which $R_1CH_2$— may represent include 1-dodecyl, 1-tetradecyl, 1-hexadecyl, 1-octadecyl and 1-eicosyl.

Examples of branched alkyl groups include 3-dodecyl, 3-tetradecyl, 3-hexadecyl, 3-octadecyl and 3-eicosyl.

Examples of suitable mono-cyclic systems include cyclodecyl and cyclododecyl. Examples of suitable bi-cyclic systems include bicyclo [2.2.1] heptyl, bicyclo [2.2.1] heptenyl, bicyclo [2.2.2] octyl and bicyclo [4.4.0] decyl. An example of a suitable polycycloalkyl group is adamantyl.

Preferably $R_1$ is an unbranched $C_{13}$ to $C_{17}$ alkyl group.

Thus a preferred class of compounds within the scope of this invention has the general formula (I) wherein $R_1$ is an unbranched $C_{13}$ to $C_{17}$ alkyl group and $R_2$ is as previously defined with reference to formula (1).

An example of a compound of this preferred class is 6-(n-hexadecylamino)-nicotinic acid.

Examples of pharmaceutically acceptable salts of the —$CO_2H$ and —$CH_2CO_2H$ include alkali metal and alkaline earth metal salts, particularly sodium, potassium, calcium and magnesium salts.

Compounds of general formula (I) also form pharmaceutically acceptable acid addition salts with appropriate acids.

Examples of suitable acids include inorganic acids such as hydrochloric, hydrobromic, hydroiodic, sulphuric, phosphoric and nitric acid and organic acids such as lower alkanoic acids (e.g. acetic and propionic), glycollic, pyruvic, malonic, succinic, malic, tartaric and citric acids.

The term pharmaceutically acceptable groups which are convertable in the human body into a —$CO_2H$ or —$CH_2CO_2H$ group means a group which is hydrolysed or is metabolised in some other way to a —$CO_2H$ or —$CH_2CO_2H$ group, and is such that the compound of formula (I) is pharmaceutically acceptable.

Groups which are convertible by hydrolysis to the —$CO_2H$ or —$CH_2CO_2H$ group are selected from pharmaceutically acceptable esters and also from terminal methyl ketones, terminal carbinols and terminal carboxylic acids (each as hereinafter defined). Examples of such esters include the $C_1$ to $C_6$ alkyl esters, in particular the methyl, ethyl and *n*-propyl esters.

Other suitable esters include for example alkoxyalkyl esters such as methoxymethyl esters; acyloxyalkyl esters such as acetoxymethyl, pivaloyloxymethyl, $\alpha$-acetoxyethyl, $\alpha$-acetoxybenzyl and $\alpha$-pivaloyloxyethyl esters; alkoxycarbonyloxyalkyl esters, such as ethoxycarbonyloxyethyl; and lactone, thiolactone and dithiolactone esters, i.e. ester groups of formula (II):—

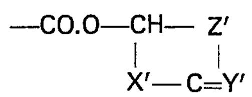

(II)

wherein X′ and Y′ are oxygen or sulphur and Z′ is an ethylene group or a 1,2-phenylene group optionally substituted by $C_1$—$C_6$ alkoxy, halogen or nitro.

Preferred ester groups are the phthalidyl and 3,4-dimethoxy phthalidyl esters.

Typical of groups which are metabolisable to $—CO_2H$ and $—CH_2CO_2H$ groups are terminal methyl ketones where $R_2$ in formula (I) represents:—

$$—(CH_2)_m.\overset{\overset{O}{\|}}{C}.CH_3$$

in which m is an integer up to 5, terminal carbinols where $R_2$ in formula (I) represents:—

$$—(CH_2)_nCH_2OH$$

in which n is zero or an integer up to 5 and terminal carboxylic acids where $R_2$ in formula (I) represents:—

$$—(CH_2)_p.CO_2H$$

in which p is an integer from 2 to 6, as well as their pharmaceutically acceptable salts and esters. Suitable pharmaceutically acceptable salts and esters of such terminal carboxylic acid containing derivatives are the same as discussed above with reference to salts and esters of the $—CO_2H$ and $—CH_2CO_2H$ groups.

Where n, m or p are odd the group is metabolised to $—CH_2CO_2H$. Where n is zero and where m, n and p are even the group is metabolised to $—CO_2H$.

When $R_2$ represents a terminal methyl ketone, m is preferably 1. An example of such a compound is 3-(2′-oxo-propyl)-6-(n-hexadecylamino)-pyridine.

When $R_2$ represents a terminal carbinol, n is preferably 1. An example of such a compound is 3-hydroxymethyl-6-(n-hexadecylamino)-pyridine.

Where $R_2$ represents a terminal carboxylic acid, p is preferably 2. An example of such a compound is 3-(6′-n-hexadeylaminopyrid-3′-yl) propanoic acid.

In order to determine whether a group $R_2$ is a group which is convertable in the human body to $—CO_2H$ or $—CH_2CO_2H$ (as required by the above description), the urine of a patient to whom the compound of formula (I) has been administered is tested to detect the presence of a compound carrying $—CO_2H$ or $—CH_2CO_2H$ at that position, and which is absent in the urine of the same patient before such administration. In some cases it may be necessary to administer the compound carrying a radioactive label on the $\alpha$-carbon in the group $R_2$, to see if a compound is excreted bearing a labelled carboxylic acid group.

Compounds of formula (I) may be prepared by reacting a halopyridine derivative with a suitable amine.

Accordingly, the invention further provides a process for preparing a compound of formula (I) which process comprises reacting a compound of formula (III):—

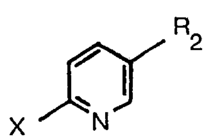

(III)

with an amine of formula $R_1CH_2NH_2$ wherein X is a halogen atom and $R_1$ and $R_2$ are defined with reference to formula (I) above, and thereafter where desired converting a compound of formula (I) so produced as a free carboxylic acid into a pharmaceutically acceptable salt or ester, converting a compound of formula (I) so produced as a salt into a pharmaceutically acceptable ester, or converting a compound of formula (I) so produced as an ester into a salt or free carboxylic acid.

Examples of suitable bases include alkali metal and alkaline earth metal carbonates and hexamethylphosphoramide. Preferably the base is a mixture of potassium carbonate and hexamethylphosphoramide.

The reaction is preferably carried out at elevated temperature i.e. greater than 100°C, 120—140°C being the most convenient temperature range.

The reaction may be carried out in the presence or absence of solvent. Generally where hexamethylphosphoramide is the base the reaction is carried out in the absence of solvent.

The time for which the reaction is allowed to proceed depends upon the nature of the starting materials and the base and the temperature at which it is carried out. Suitable reaction times may be determined by following the course of reaction with any standard technique such as thin layer chromatography. Generally, under the preferred conditions referred to above, the reaction is complete within 3 to 5 hours.

The compounds of this invention may also be prepared by alkylating an aminopyridine with an alkyl halide.

Accordingly the present invention provides a process for preparing a compound of formula (I) above which process comprises reacting a compound of formula (IV):—

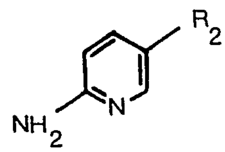

(IV)

with an alkyl halide $R_1X$, wherein $R_1$ and $R_2$ are as defined with reference to formula (I) and X is halogen in the presence of a base, and thereafter where desired converting a compound of formula (I) so produced as a free carboxylic acid into a pharmaceutically acceptable salt or ester, or converting the compound of formula (I) so produced as a salt into a free acid or ester, or converting a compound of formula (I) so produced as an ester into a free acid or salt.

This process is carried out in a manner analogous to that described above with reference to compounds of formula (III).

Compounds of formula (I) above may also be prepared by reducing a corresponding enamine (V):—

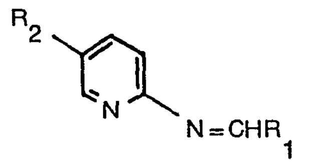

(V)

wherein $R_1$ and $R_2$ are defined with reference to formula (I) with a reducing agent, and thereafter where desired converting a compound of formula (I) so produced as a free carboxylic acid into a pharmaceutically acceptable salt or ester, or converting the compound of formula (I) so produced as a salt into a free acid or ester, or converting a compound of formula (I) so produced as an ester into a free acid or salt.

A suitable reagent to effect this reduction is a metal hydride such as sodium borohydride in an alcoholic solvent, particularly ethyl alcohol. Alternatively, catalytic hydrogenation may be employed, for example using a platinum oxide catalyst.

The intermediates of formula (V) may be prepared by reaction of an amine of formula (VI) with an aldehyde of formula (VII):—

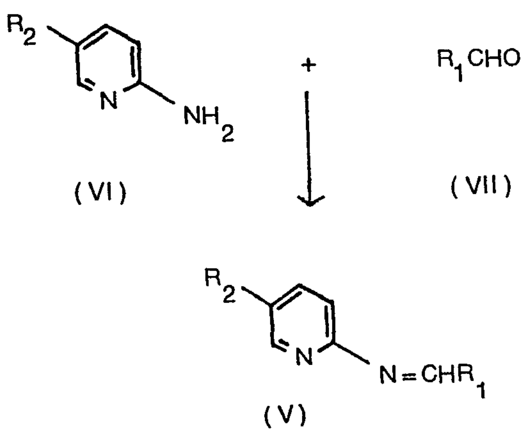

(VI) (VII) (V)

It may be convenient to generate the intermediate (V) *in situ*, by reacting an amine of formula (VI) with an aldehyde of formula (VII) in the presence of a reducing agent. In such a case, a suitable reducing agent is hydrogen on a platinum oxide catalyst in a suitable solvent such as a mixture of methanol and dioxan.

Compound of formula (I) may also be prepared by reducing a compound of formula (VIII):—

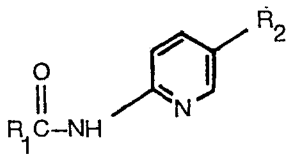

(VIII)

wherein $R_1$ and $R_2$ are as defined with reference to formula (I) and thereafter where desired converting a compound of formula (I) so produced as a free carboxylic acid into a pharmaceutically acceptable salt or ester or converting a compound of formula (I) so produced as a salt into a free acid or ester, or converting a compound of formula (I) so produced as an ester into a free acid or salt.

A suitable reagent for reducing compound (VIII) is borane in dimethyl sulphide.

Intermediates (VIII) may be prepared by acylation of a compound (VI) as defined above in a conventional manner.

Compounds (I) produced as free acids or salts in the above processes may be converted to esters by conventional methods. Similarly esters and salts may be converted into free acids and salts may be converted to esters by conventional methods.

In order to use the compounds of formula (I) as hypolipidaemic agents they may be formulated as pharmaceutical compositions in a variety of dosage forms.

Accordingly, in a further aspect, the invention provides a pharmaceutical composition comprising a compound of formula (I) as defined above or a pharmaceutically acceptable salt or ester thereof together with a pharmaceutically acceptable carrier.

The compositions may be formulated for systemic administration by any route, although oral administration is preferred. The compositions may be in the form of tablets, capsules, powders, granules, lozenges or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinyl-pyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example, sorbitol, methyl cellulose, glucose syrup, gelatin, hydroxyethylcellulose, carboxymethylcellulose, aluminium stearate gel or hydrogenated edible fats, emulsifying agents, for example lecithin, sorbitan monooleate, or acacia, non-aqueous vehicles (which may include edible oils) for example almond oil, fractionated coconut oil, oily esters such as glycerine, propylene glycol, or ethyl alcohol; preservatives, for example methyl or propyl *p*-hydroxybenzoate or sorbic acid; and if desired conventional flavouring or colouring agents.

Suppositories will contain conventional suppository bases, e.g. cocoa butter or other glycerides.

For parenteral administration, fluid unit dosage forms are prepared utilizing the compounds and a sterile vehicle, water being preferred. The compound, depending on the vehicle and concentration used, can be either suspended or dissolved in the vehicle. In preparing solutions the compound can be dissolved in water for injection and filter-sterilized before filling into a suitable vial or ampoule and sealing. Advantageously, adjuvants such as local anaesthetic, preservative and buffering agents can be dissolved in the vehicle. To enhance the stability, the composition can be frozen after filling into the vial and the water removed under vacuum. The dry lyophilized powder is then sealed in the vial. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The composition may contain from 0.1% to 99% by weight, preferably from 10—60% by weight, of the active material, depending on the method of administration. Where the compositions comprise dosage units, each unit will preferably contain from 250 mg—3 g, of the active ingredient. The dosage as employed for adult human treatment will preferably range from 1 to 10 g, per day, for instance 3 g, per day, depending on the route and frequency of administration.

The following examples illustrate the invention:—

Example 1

Preparation of methyl-6-(n-hexadecylamino)-pyridine-3-carboxylate

Methyl-6-chloropyridine-3-carboxylate (3.44 g; 0.02 m) was added to a mixture of n-hexadecylamine (6.05 g; 0.025 m) and anhydrous potassium carbonate (6 g) in hexamethylphosphoramide (30 ml). The mixture was heated at 130°C for 90 minutes with vigorous stirring, cooled to room

temperature, and water (150 ml) was added. The solution was extracted with dichloromethane (2 x 100 ml) and the organic extracts were dried ($MgSO_4$) and evaporated to dryness. The residual oil was dissolved in diethyl ether (100 ml), washed with water (2 x 100 ml), and the organic phase was dried ($MgSO_4$) and evaporated to give 6.9 g of solid product. Crystallisation of the product from ether yielded methyl-6-(n-hexadecylamino)-pyridine-3-carboxylate (3.9 g; 52%) M.P. 88—90°C.

| Analysis | | Req. | Found |
|---|---|---|---|
| | C | 73.40 | 73.09 |
| | H | 10.64 | 10.82 |
| | N | 7.45 | 7.32 |

Example 2

Preparation of 6-(n-hexadecylamino)-pyridine-3-carboxylic acid hydrochloride

Methyl-6-(n-hexadecylamino)-pyridine-3-carboxylate (7.52 g; 0.02 m) in methanol (20 ml) was treated with potassium hydroxide (5.6 g; 0.1 m) in water (20 ml) and the mixture was boiled under reflux with stirring for 90 minutes. The product was cooled, the solid filtered, washed with water (50 ml) and dried to give the potassium salt. This salt was suspended in 10% hydrochloric acid solution (100 ml) and boiled under reflux with stirring for 1 hour. The solution was cooled, diluted with water (200 ml), filtered, and the solid product dried under vacuum to give 6-(n-hexadecylamino)-pyridine-3-carboxylic acid hydrochloride (5.1 g; 64%)
M.P. — 150°C Decomp.

| Analysis | | Req. | Found |
|---|---|---|---|
| | C | 66.25 | 67.00 |
| | H | 9.78 | 9.92 |
| | N | 7.02 | 6.94 |

**Claims**

1. A compound of formula (I):

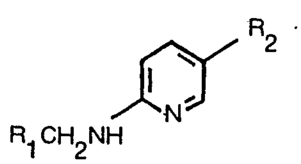

(I)

wherein $R_1CH_2$ is a $C_9$ to $C_{25}$ alkyl or alkenyl group which is optionally branched or unbranched or includes a mono-, bi- or polycycloalkyl or —cycloalkenyl moiety and $R_2$ represents a $—CO_2H$ or $—CH_2CO_2H$ group or a pharmaceutically acceptable salt thereof or a pharmaceutically acceptable group, which is convertible in the human body into a $—CO_2H$ or $—CH_2CO_2H$ group, selected from a pharmaceutically acceptable ester

$$-(CH_2)_m-\underset{\underset{O}{\|}}{C}-CH_3 \qquad -(CH_2)_n-CH_2OH \qquad -(CH_2)_p-CO_2H$$

where m is an integer up to 5, n is zero or an integer up to 5 and p is an integer from 2 to 6.

2. A compound as claimed in claim 1 wherein the group $R_1CH_2$— contains not more than 20 carbon atoms.

3. A compound as claimed in claim 1 wherein $R_1$ represents an unbranched $C_{13}$ to $C_{17}$ alkyl group.

4. 6-(n-Hexadecylamino)-pyridine-3-carboxylic acid, or a $C_{1-6}$ alkyl ester thereof, or a pharmaceutically acceptable acid addition salts of the acid or ester thereof.

5. A process for the preparation of a compound as claimed in claim 1, which process comprises:

a) reacting a compound of formula (III):

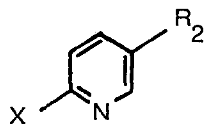

(III)

with an amine of formula $R_1CH_2NH_2$ wherein X is a halogen atom and $R_1$ and $R_2$ are as defined in claim 1: or
b) reacting a compound of formula (IV):

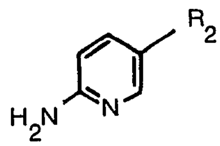

(IV)

with an alkyl halide of formula $R_1X$, wherein $R_1$ and $R_2$ are as defined in claim 1 and X is a halogen, in the presence of a base; or
c) reducing an enamine of formula (V) or an amide of formula (VIII):

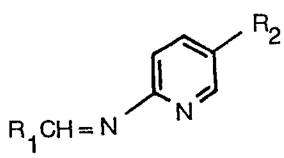

(V)

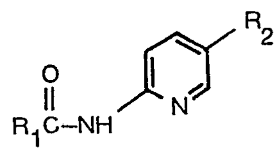

(VIII)

wherein $R_1$ and $R_2$ are as defined in claim 1, with a reducing agent;
and after processes a), b) or c), then optionally converting a compound of formula (I) so produced as a free carboxylic acid into a pharmaceutically acceptable salt or ester, converting a compound of formula (I) so produced as a salt into a free acid or a pharmaceutically acceptable ester, or converting a compound of formula (I) so produced as an ester into a salt or free carboxylic acid.

6. A pharmaceutical composition which comprises a compound as claimed in claim 1 together with a pharmaceutically acceptable carrier.

**Revendications**

1. Composé de formule (I):

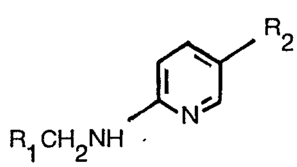

(I)

dans laquelle $R_1CH_2$ est un groupe alcoyle ou alcényle en $C_9$ à $C_{25}$ qui est à volonté ramifié ou non ramifié ou qui comporte une fraction molaire cycloalcoyle ou cycloalcényle, et $R_2$ représente un groupe $—CO_2H$ ou $—CH_2CO_2H$ ou un sel pharmaceutiquement acceptable de celui-ci, ou bien un groupe pharmaceutiquement acceptable qui peut être converti dans le corps humain en un groupe $—CO_2H$ ou $—CH_2CO_2H$.

2. Composé suivant la revendication 1, dans lequel le groupe $R_1CH_2—$ ne contient pas plus de 20 atomes de carbone.

3. Composé suivant la revendication 1, dans lequel $R_1$ représente un groupe alcoyle non ramifié en $C_{13}$ à $C_{17}$.

4. Acide 6-(n-hexadécylamino)-pyridine-3-carboxylique ou ester d'alcoyle en $C_{1-6}$ de celui-ci, ou bien sel d'addition avec un acide pharmaceutiquement acceptable de l'acide ou de son ester.

5. Procédé pour la préparation d'un composé suivant la revendication 1, ce procédé consistant:

a) à faire réagir un composé de formule (III):

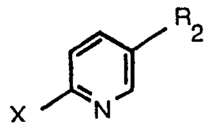

(III)

avec une amine de formule $R_1CH_2NH_2$ dans laquelle X est un atome d'halogène et $R_1$ et $R_2$ ont la même signification que dans la revendication 1; ou

b) à faire réagir un composé de formule (IV):

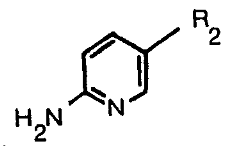

(IV)

avec un halogénure d'alcoyle de formule $R_1X$, dans laquelle $R_1$ et $R_2$ ont la même signification que dans le cas de la formule (I) et X est un halogène, en présence d'une base; ou

c) à réduire une énamine de formule (V) ou bien un amide de formule (VIII):

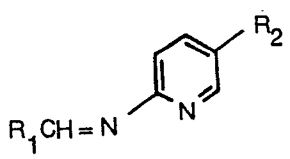

(V)

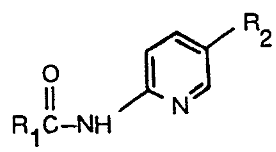

(VIII)

dans lesquelles $R_1$ et $R_2$ ont la même signification que dans le cas de la revendication 1 avec un agent réducteur; et après les procédés a), b) ou c) à convertir éventuellement un composé de formule (I) ainsi produit sous forme d'un acide carboxylique libre en un sel ou ester pharmaceutiquement acceptable, à convertir un composé de formule (I) ainsi produit sous forme d'un sel en un acide libre ou en un ester pharmaceutiquement acceptable, ou à convertir un composé de formule (I) ainsi produit sous forme d'un ester en un sel ou en un acide carboxylique libre.

6. Composition pharmaceutique renfermant un composé suivant la revendication 1, conjointement à un véhicule ou excipient pharmaceutiquement acceptable.

**Patentansprüche**

1. Verbindung der Formel (I)

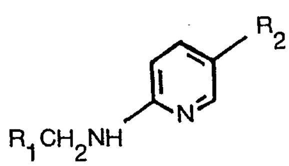

(I)

in der $R_1CH_2$ ein Alkyl- oder Alkenylrest mit 9 bis 25 Kohlenstoffatomen ist, der gegebenenfalls verzweigt oder unverzweigt ist oder einen Cycloalkyl- oder Cycloalkenylteil enthält, und $R_2$ die —COOH— oder die —$CH_2COOH$— Gruppe oder deren pharmakologisch verträgliche Salze bedeutet, die im menschlichen Körper in die —COOH— oder —$CH_2COOH$-Gruppe überführbar ist.

2. Verbindung nach Anspruch 1, in der der Rest $R_1CH_2$— nicht mehr als 20 Kohlenstoffatome enthält.

3. Verbindung nach Anspruch 1, in der $R_1$ ein unverzweigter Alkylrest mit 13 bis 17 Kohlenstoffatomen ist.

4. 6-(n-Hexadecylamino)-pyridin-3-carbonsäure oder deren $C_{1-6}$-Alkylester oder ein pharmakologisch verträgliches Säureadditionssalz der Säure oder des Esters.

5. Verfahren zur Herstellung einer Verbindung nach Anspruch 1, wobei das Verfahren die Maßnahmen umfaßt:

a) Umsetzen einer Verbindung der Formel (III)

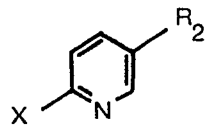

(III)

mit einem Amin der Formel $R_1CH_2NH_2$, in denen X ein Halogenatom ist und $R_1$ und $R_2$ die in Anspruch 1 angegebenen Bedeutungen besitzen; oder

b) Umsetzen einer Verbindung der Formel (IV)

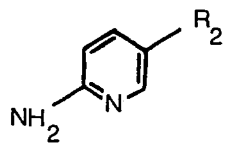

(IV)

mit einem Alkylhalogenid der Formel $R_1X$, in denen $R_1$ und $R_2$ die in Anspruch 1 angegebenen Bedeutungen besitzen und X ein Halogenatom ist, in Gegenwart einer Base; oder

c) Reduzieren eines Enamins der Formel (V) oder eines Amids der Formel (VIII)

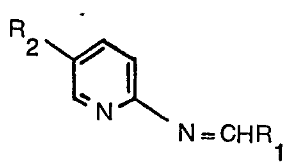

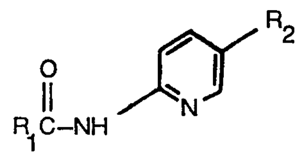

(VIII)

(V)

in denen $R_1$ und $R_2$ die in Anspruch 1 angegebenen Bedeutungen besitzen, mit einem Reduktionsmittel;

und nach den Verfahrensschritten a), b) oder c) gegebenenfalls eine derart als freie Carbonsäure hergestellte Verbindung der Formel (I) in eine Salz oder einen Ester, die pharmakologisch verträglich sind, oder eine derart als Salz hergestellte Verbindung der Formel (I) in eine freie Säure oder einen pharmakologisch verträglichen Ester oder eine derart als Ester hergestellte Verbindung der Formel (I) in ein Salz oder freie Carbonsäure überführt.

6. Pharmazeutische Zubereitung, die die Maßnahme einer Verbindung nach Anspruch 1 zusammen mit einem pharmakologisch verträglichen Trägermaterial umfaßt.